Europäisches Patentamt

European Patent Office

Office européen des brevets

(11) Publication number: **0 419 662 A1**

# EUROPEAN PATENT APPLICATION
## published in accordance with Art. 158(3) EPC

(21) Application number: 89909896.6

(22) Date of filing: 18.04.89

(86) International application number:
PCT/SU89/00099

(87) International publication number:
WO 90/12558 (01.11.90 90/25)

(51) Int. Cl.⁵: **A61H 39/00, A61N 5/02,
A61N 1/40**

(43) Date of publication of application:
**03.04.91 Bulletin 91/14**

(84) Designated Contracting States:
**AT BE CH DE FR GB IT LI NL SE**

(71) Applicant: VREMENNY NAUCHNY KOLLEKTIV "OTKLIK"
ul. Vladimirskaya, 61b
Kiev, 252017(SU)

(72) Inventor: SITKO, Sergei Panteleimonovich
ul. Semenovskaya, 11-64
Kiev, 252110(SU)
Inventor: LOBAREV, Valery Evgenievich
ul. Marshala Grechko, 12-79
Kiev, 252136(SU)
Inventor: KOLBUN, Nikolai Dmitrievich
ul. Kalinina, 14-9/3
Kiev, 252001(SU)

(74) Representative: Wain, Christopher Paul et al
A.A. THORNTON & CO. Northumberland
House 303-306 High Holborn
London WC1V 7LE(GB)

(54) DEVICE FOR MICROWAVE RESONANCE THERAPY.

(57) The device comprises a source of electromagnetic oscillations in the millimeter wave band, for example a noise generator (1) whose output is connected to a radiating aerial (2). The source of oscillations provides for a continuous spectrum of oscillations over the therapeutically efficient frequency range with a spectral power density ranging from $10^{-6}$ W/Hz to the quantum limit. The invention may be used for treating patients suffering from diseases of different etiology, including disorders of the gastroduodenal and emotional-motivational spheres, chronic pulmonary diseases, infantile cerebral paralysis and other diseases.

FIG.1

# DEVICE FOR MICROWAVE RESONANCE THERAPY

## Technical Field

The invention relates to treatment of diseases by virtue of contactless effect upn biologically active zones produced by EHF electromagnetic fields, more specifically to a device for microwave resonance therapy.

## Background Art

It is established presently that the effect of low-(nonthermal)-intensity radiations at a number of fixed millimetric wave band (EHF radiations) contributes to elimination of dysfunctioning of human organism and to preparation of the latter for resistance to toxic effect of chemical medicinal agents and ionizing radiations. The essence of the effect produced by the EHF radiations on human organism is determined by the fact that the organism's cells themselves generate signals instrumental in managing a process of restoration or adaptation to the varied living conditions. Should the generation of such signals be upset or retarded either due to diseases undergone or on account of advanced age, their deficiency may be compensated for by the use of similar signals generated by external sources. Electromagnetic radiation of a millimetric wave band having low-intensity radiation power ( $\leqslant$ 10 mW/cm$^2$) have found most utility when applied for medicobiological studies or in treatment of certain diseases.

One prior-art method of microwave resonance resonance therapy (SU, A, 1,341,762) is known to consist in that effect is produced on a human organism by low-power EHF radiation (power flow density being within 0.01 and 10 mW/cm$^2$) in the acupuncture areas at a fixed individually selected frequency range from 44 to 70 GHz. First the wave frequency is varied steplessly in said range until a stable sensory reaction in the radiation zone, whereupon further treatment is is carried out with the aforesaid parameters until the sensory reaction in the radiation zone disappears.

The known method is substantially disadvantageous in too long a time required (2 to 4 hours, and even more) for individual selection of a fixed frequency, which extends the treatment period and increases the energy level of action produced by electromagnetic radiation on human organism.

Besides, selection of a fixed frequency in accordance with patient's complex of sensations is a subjective procedure.

One state-of-the-art device for microwave resonance therapy (cf. "Appliances for microwave reflexodiagnosis and reflexotherapy "Elektronika-KVTch' by A.N.Balaba et al. In: Elektronnaya promyshlennost, Issue 1(159), 1987, p. 30 (in Russian) is known to comprise an EHF-band electromagnetic wave source whose output is connected to a radiating antenna. The source of electromagnetic waves is essentially a generator provided with an electrically controlled attenuator, which along with a detecting device enables one to steplessly adjust the level of the output radiation power, to stabilize said output power, as well as to carry out continuous-wave generation and amplitude modulation modes either separately or in combination with frequency modulation.

All disadvantages of the aforediscussed known method are inherent in the device described above.

## Summary of the Invention

It is a primary and essential object of the present invention to provide a device for microwave resonance therapy, wherein the electromagnetic radiation source would feature such a construction that provides for automatic selection of the EHF-band frequencies corresponding to the characteristic frequencies of patient's biologically active zones with the purpose of exerting a resonance effect on said zones.

Said object is accomplished due to the fact that in a device for microwave resonance therapy, comprising an EHF-band electromagnetic wave source whose output is connected to a radiating antenna, according to the invention, used as a source of electromagnetic oscillations is a source of continuous-frequency spectrum oscillations in a therapeutically effective frequency band having a spectral power density of from 10$^{-6}$ W/Hz to a value approximating the quantum limit.

It is quite expedient that use be made of a source of oscillations having a spectral power density of electromagnetic radiation within 10$^{-16}$ and 10$^{-18}$ W/Hz.

According to one of the embodiments of the invention used as a source of electromagnetic oscillations is a noise generator.

In an alternative embodiment of the invention the noise generator comprisesa a shaper of supply voltage connected to the input of an active element, and a means for channelling of electromagnetic energy emitted by the active element, while a radiating antenna is connected to the output of said means for channelling of electromagnetic energy.

In one more alternative embodiment of the invention the means for channelling of electromagnetic energy is in effect a waveguide adapted for low-pass filtering of electromagnetic radiation.

And according to a further alternative embodiment of the invention the device comprises additionally a waveguide transformer, whereby communication is established between the means for channelling of electromagnetic energy and the radiating antenna.

Therapeutic modality produced with the aid of the proposed device microwave resonance therapy is distinguished for quick action, thus cutting down the treatment time as compared not only with the tradiation therapy methods but also with the heretofore-known techniques of microwave resonance therapy, at the same time providing a stable therapeutic effect. Besides, the treatment process is substantially simplified, while labour consumption and time spent for carrying out treatment sessions are cut down due to dispensing with the resonance frequency selection procedure.

For the same reason one may consider the treatment process as more efficatious, since the patient's organism itself is free to "choose" those frequencies in the radiation frequency band at which occurs resonance interaction of patient's organism with an external electromagnetic radiation applied to biologically active areas. In such a situation the organism gets incorporated into a system of correction of its state as an active element.

The device described herein makes it possible to do away with the use of chemotherapy and as such rules out any negative effect on patient's organism. The fact is corroborated by the findings of clinical trials.

The device of the invention is applicable both under outpatient conditions and in a hospital; when used in rendering medical aid under patient's domiciliary conditions the device is conducive to considerably cut down the treatment costs.

It is worth noting that when applied for treatment of the cases of infantile cerebral palsy the proposed device gives a better therapeutic effect than the known means of medicinal, functional, phsiotherapeutic, health-resort, or other kinds of treatment.

One more important advantage of the proposed device resides in efficacious treatment of patologies affecting the emotional-motivation field.

Of paramount importance is such an advantage of the proposed device as an unprecedentedly low energy level of the effect produced, that is, by at least six orders of magnitude lower as compared with the now-adopted value in the existing practice of microwave resonance therapy.

Brief Description of the Drawings

Further objects and advantages of the present invention will become evident from the following detailed description of some exemplary embodiments thereof with reference to the accompanying drawings, wherein:

FIG. 1     is a block diagram of a device for microwave resonance therapy, according to the invention;

FIG. 2     is at elementary electric diagram of a device, according to the invention; and

FIG. 3     is a block diagram of an alternative embodiment of a device, according to the invention.

Best Mode of Carrying Out the Invention

The method for microwave resonance therapy is in fact as follows. The EHF-band low-intensity electromagnetic radiation is applied to the biologically active zones and areas on human body (such as acupuncture points, hyper- and hyposensitive areas, Head's zones, the scalp, the planter surface, and others), which are associated with, and characteristic of, the treatment of any specific pathology, said radiation being in fact continuous-spectrum oscillations in a therapeutically effective frequency band. The electromagnetic radiation source is also featured by a spectral power density ranging within $10^{-6}$ W/Hz to the value approximating the quantum limit and equal to about $10^{-21}$ W/Hz.

The duration of a treatment session is selected within half an hour; it depends on a plurality of factors, such as individual patient's individual susceptibility, the stage and severity of the disease, general patient's physiological and hormonal status, the kind of pathology, and the like.

Taking due account of the prerequisites mentioned above an important parameter of the radiation source is a time-average spectral density of electromagnetic radiation power, which is maintained preferably within the limit of from $10^{-16}$ to $10^{-18}$ W/Hz. Treatment experience shows that a curative effect is attained within a treatment course comprising from 1 to 15 sessions, preferably 7 to 15 sessions.

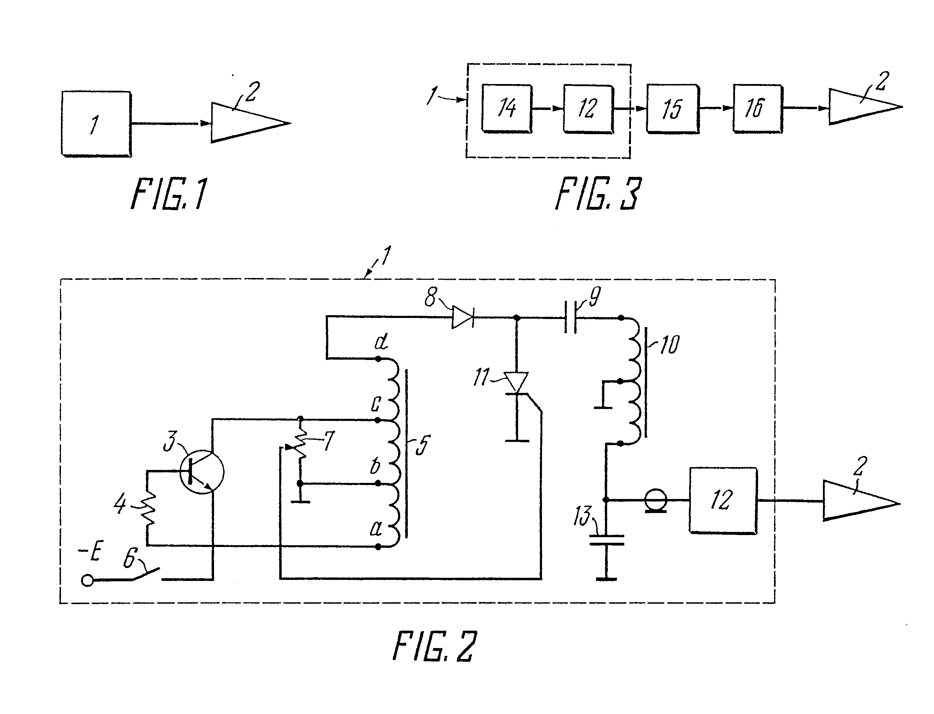

The device for microwave resonance therapy comprises an EHF-band noise generator 1 (FIG. 1) aimed at generating continuous-spectrum electromagnetic oscillations, its output being connected to a radiating antenna 2, which shapes a directional flow of electromagnetic radiation for irradiating a preselected biologically active zone of patient's skin surface.

The generator 1 comprises a supply voltage shaper connected in a blocking-oscillator circuit and incorporating a transistor 3 (FIG. 2), a resistor 4 cut in the base circuit of the transistor 3 and connected to a tap 'a' of the winding of a pulsing transformer 5 whose tap 'c' is connected to the collector of the transistor 3. The emitter of the transistor 3 is connected to the negative terminal of a supply voltage source E through a switch 6. The collector of the transistor 3 is connected to a common bus and to a tap 'b' of the pulsing transformer 5 through a variable resistor 7. A tap 'd' of the pulsing transformer 5 is connected to the anode of a diode 8, whose cathode is connected to a reservoir capacitor 9 series-connected to the coil of a step-up autotransformer 10. A thyristor 11 serving as a switching element is connected, through its anode, to the cathode of the diode 8, and through its cathode, to the commone bus. The control electrode of the thyristor 11 connected to the centretap of the resistor 7. The secondary tap of the autotransformer 10 is connected to the positive terminal of an active element 12 of the noise generator 1 in the capacity of which use may be made of an IMPATT diode, a semi-conductor photodiode, a gaseous-discharge lamp, an avalanche transistor, a lambda-diode, a tunnel diode, or a backward diode. Depending on the type of the active element 12 a filter capacitor 13 is selected. The aforedescribed construction of the device for microwave resonance therapy is instrumental in establishing at its output an electromagnetic noise radiation in a continuous spectrum of wavelengths of from 1 to 10 mm featuring a spectral power within the range of from $10^{-6}$ W/Hz to a value approximating the quantum noise limit in the EHF band.

FIG. 3 presents an alternative embodiment of the device, wherein the generator 1 comprises a supply voltage shaper 14 connected to the input of the active element 12, while connected to the output of the active element 12 is a means 15 for channelling of electromagnetic energy emitted by the active element 12. Said means 15 may be shaped as a hollow or dielectric waveguide adapted for low-pass filtering of electromagnetic radiation, and a microstrip transmission line. The output of the means 15 is connected to the radiating antenna through a waveguide transformer 16 aimed at matching the wave impedances of said means 15 and said radiating antenna 2.

The device for microwave resonance therapy operates as follows.

Inasmuch as the electromagnetic radiation generated by the device features, according to the invention, a low value of spectral power density, e.g., $10^{-18}$ W/Hz, so a technologically simple solution of the noise generator 1 is possible. The device can be supplied from a low-power source having a voltage of, e.g., from 2.5 to 5.5 V, which can be boosted, by a voltage converter, to a value required for the normal operation of the active element 12 of the noise generator 1, e.g., 2 kV for the gaseous-discharge lamp-based active element 12, or 20 V for an IMPATT diode.

Once the power supply source E has been connected, through the switch 6, to the emitter cf the transistor 4 of the blocking-oscillator, high-amplitude positive-polarity voltage pulses appear at the tap 'd' of the pulsing transformer 5, said pulses having a repetition frequency of a few kilohertz and charging the reservoir capacitor 9 until such a voltage appears on the anode of the thyristor 11 that a next voltage pulse sent by the voltage divider based on the resistor 7 to the control electrode of the thyristor 11 drives the latter to conduction, whereupon the reservoir capacitors 9 discharges, through the thyristor 11, into the winding of the step-up autotransformer 10. During said discharge a high-voltage pulse is shaped in the secondary of the autotransformer 10, which is then impressed on the active element 12 of the noise generator 1, which establishes electromagnetic oscillations in the EHF band of wavelengths. The thus-established electromagnetic field is emitted through the radiating antenna 2, into the surrounding atmosphere. The frequencies of discharge of the reservoir capacitor 9 is set up with the aid of the variable resistor 7. The filter capacitor 13 is used whenever it is necessary to smooth out voltage fluctuations of the active element 12 of the noise generator 1.

To exert therapeutic effect the antenna 2 is brought as close as 5 to 20 mm to the patient's biologically active zone preselected for treatment of a given pathology.

In what follows the essence of the proposed method is illustrated by some examples of its practical application involving the use of the proposed device with reference to specific kinds of pathology.

Example 1

Treatment of duodenal ulcer patients

The method was applied to a total of 94 patients of both sexes suffering from peptic ulcer of the duodenum at the stage of exacerbation and with the developed "niche". The age bracket of the treated patients was within 12 and 60, the duration of the disease, from 1 to 25 years.

The treatment was carried out as follows. The EHF-band low-intensity electromagnetic radiation was applied to the biologically active zones, that is, acupuncture points associated with a given pathology, said radiation featuring a continuous spectrum in a therapeutically effective EHF waveband, i.e.,

from 1 to 10 mm and a spectral power density within $10^{-6}$ and $10^{-18}$ W/Hz. It is worth noting that a therapeutic effect was obtained with lower values of spectral power density approximating the quantum limit (down to $10^{-21}$ W/Hz).

The treatment effect was produced with the aid of the aforesaid device for microwave resonance therapy in the course of a few sessions, that is, from 7 to 15 per patent. The duration of a session was 25 to 30 minutes daily.

To take an example, male patient A., 45 was treated by the method described above, the diagnosis being one of peptic ulcer of the duodenum accompanied by pain syndrome and lasting more than 14 years.

Within the recent 9 or 10 years painful periods became still more frequent, then painful syndrome got permanent and was of the same nature for the recent two years. The patient had early been given a course of general-therapy treatment which however failed to abolish pain syndrome. Objective examination methods with the use of Ro-scopy and fibrogastroduodenoscopy detected the presence of the duodenal bulb with an ulcer defect ij the bulb wall measuring 0.8x1.5 cm. The treatment course incorporated 10 sessions performed daily and taking 25 minutes each. In the course of treatment the patient had a sensation of pulsating heat in the abdominal cavity accompanied by a sensation of massage in the same cavity, and drowsiness. The pain syndrome was eliminated by the end of the treatment course. No negative side effects were observed. Fibrogastroduodenoscopy revealed the presence of a scar at the place of the ulcer. No defects of the mucosa were present.

Given below are data characteristic of the treatment efficiency, said data being corporated by fibrogastroduodenoscopy.

Complete healing of the ulcer defect was observed in 84 patients (89.4 percent) of whom:

in 8 patients after 7 sessions;
in 9 patients after 8 sessions;
in 17 patients after 9 sessions;
in 41 patients after 10 sessions;
in 2 patients after 14 sessions; and
in 7 patients after 15 sessions.

In other 9 patients (9.6 percent) there was observed positive dynamics in healing of the ulcer defect, that is, the ulcer having healed by more than half after:

10 sessions in 7 patients;
15 sessions in 2 patients.

One patient in said group (1 percent) exhibited no positive dynamics in healing of the ulcer defect after 11 sessions.

Example 2

Treatment of patients suffering from chronic non-specific pulmonary diseases and bronchus-obstructive syndrome

Treatment of patients with the aforesaid pathologies was carried out by the proposed method in a way similar to that described in Example 1, with the sole exception that control of the treatment efficacy was carried out by electrocardiography and roentgenography of the chest, using the findings of general clinical and biochemical analyses of the blood and urine.

A total of 24 patients were given the treatment course. As a result, in patients with bronchial asthma and chronic obstructive bronchitis the obstructive syndrome was abolished rapidly and the respiratory function was ameliorated, while in some of the patients dependence of the intensity of a pathologic process on the hormonal status was eliminated.

Male patient K., 39 of said group was treated by the proposed method, the diagnosis being one of bronchial asthma in an infection-allergic form, status asthmaticus. The patient has been suffering for 14 years. The known treatment method, such as acupuncture and electropuncture were of no effect. The patient was given a complex of broncholytics, hormonal preparations, intravenous infusions, inhalations. Attacks of dyspnea occurred daily.

The patients was exposed to the effect of electromagnetic radiation in the wavelength range of 5 to 5.5 mm with a spectral power density within $10^{-16}$ to $10^{-18}$ W/Hz applied for 20 minutes to the biologically active acupuncture zones specified by the cartography of reflexo therapy. The following clinical manifestations of the treatment were observed: sensations of aspiratorily embrarrassed breathing relieved, inhaling was shortened, exhaling was normalized, the amount of stertors diminished, the dyspnea phenomena were abolished, the respiratory rate and cardiac rhythm were normalized.

The treatment course was comprised of 9 sessions. The attacks of dyspnea ceased completely as early as after the second procedure, while stable normalization of the external respiration functions, according to spirogram evidence and that of the Votchal-Tiffnaud's test, set in by the 9th day. Control of the treatment efficiency was carried out thrice for the six-month period that followed, the patient's general state being satisfactory.

Example 3

Treatment of patients with postthydrotoxic encephalophthalmopathy

Treatment of patients suffering from postthyrotoxic encephalophthalmopathy was carried out largely in same way as described in Example 1

with the sole exception that control was effected by immunological and biochemical methods.

A total of 12 patients were subjected to a treatment course. The treatment efficiency was characterized by the fact that for 8 to 10 sessions headache was abolished completely, sleep was improved, lancinating pain in the eyes disappeared, exophthalm os was diminished, and patients' working capacity was increased.

Female patient M., 30 incorporated in said group was treated by the proposed method. Upon admission to the clinic the patient complained of headache, dizziness, lancinating pain the eyes, exophthalmos, sensation of a foreign body in the eyes, photophobia, edema of the periorbital tissues, and diplopia. Before arrival at the clinic the patient had sustained subtotal resection of the thyroid gland for diffuse toxic goiter. After surgery some ophthalmopathic and neurologic symptoms appeared, such as bulging eyeballs, edema and hyperemia of the ocular mucosa, retraction of the upper lids, discordant motions of the eyeballs and the upper lids, reduction of some abdominal reflexes and increase of the tendon reflexes, disturbed ovarian-menstrual cycle, labile arterial blood tension, and hyperhidrosis. Conservative treatment given to the patient proved to be futile.

The patient was exposed to the effect of electromagnetic radiation in the wavelength range of 5 to 5.5 mm with a spectral power density within $10^{-12}$ to $10^{-15}$ W/Hz., applied to the biologically active zones $T_{20}$, $VB_1$, $GL_4$. Duration of a single procedure (exposure) was 25 minutes daily, a total treatment course consisting of 8 sessions.

Upon completion of the treatment course headache disappeared, as well as lancinating pain in the eyes, exophthalmos, hyperemia of the ocular mucosa, the reflexes were normalized.

The patient was examined in a clinic three times for a six-month posttreatment period, no complaints being made. No symptomocomplex of postthyrotoxic encephalohthalmopathy was observed. A trend towards abolishment of oculopathic and neurologic symptoms for a one-month posttreatment period was obvious.

Example 4

Treatment of patients with infantile cerebral palsy

The method was carried out mainly in the same way as in Example 1 with reference to patients suffering from infantile cerebral palsy in the form of spastic diplegia. Infant patients of different age were subjected to the treatment by the proposed method, the general status of whom was characterized predominantly by motor dysfunctions. Among the causes of such disturbances it is worth noting the effect of reduced topic reflexes, pathologic conditions and myogenic contractures of the upper and lower limbs, or the presence of pathologic reflexes and synkinetic phenomena. Some of the patients suffered also from speech disturbances in the form of spastico-paretic dysarthria.

In the course of the treatment course, both in the form of a single procedure and during repeated sessions, all the infant patients exhibited reduced level of bioelectric activity in spastically tensioned muscles and their synergists and improved spectral characteristics and reciprocal interaction. There were also observed normalized vestibulometric parameters, preponderantly those regulated at the mesencephalic and cortical levels, i.e., the higher regulation levels, accompanied by smoothed-out intercochlear asymmetry and improved interaction of the statoliths and the semicircular canals. Normalization of the paraclinical characteristics was accompanied, to a certain extent, by improvement of the motor function.

Efficiency of treatment by the method and device proposed, according to the invention, using global electromyography of the muscles of the upper and lower limbs and involving analysis into phase-amplitude characteristics in the course of treatment and assessment of reciprocal conjugation. These were also employed vestibulometry methods involving isolation of level organization of preferable normalization of neurodynamic characteristics.

Thus, a total of 40 patients with infantile cerebral palsy were subjected to treatment by the present method, the patients' age bracket being within 1 and 15. The group covered infant patients with moderately severe and severe stages of the disease, as well as those patients in whom there was absent the speech contact due to too early age or on account of bad psychic derangements.

A treatment course covered 10 sessions lasting 20 minutes each. Whenever it became necessary the treatment course was repeated a month later. The treatment was given with the aid of the device mentioned above using electromagnetic radiation in the wavelength range of 5 to 5.5 mm within a continuous frequency spectrum in said band with a spectral density of radiation power within $10^{-18}$ to $10^{-16}$ W/Hz.

The following biologically active points were selected for application of said radiation: acupuncture points, hyper- or hyposensitivity areas, Head's zones, the scalp, the plantar surface, and others.

As early as by the 10th or 15th second the patients experienced relaxation sensations.

The following clinical appearances were observed in the course of the treatment period: reduced pathological muscular hypertonia, higher scope of movements in the affected joints, rise of strength in paretic muscles, reduced frequency and less pronouncedness of involuntary forced movements, improved coordination of movements, and better posture. The patients exhibited better adequacy to external stimuli, normalized sleep, appetite, extended vocabulary, and speech articulation became more distinctive. Patients' fatiguability was decreased so that the children could walk for a distance several times longer than before treatment.

Example 5

Treatment of patients suffering from chronic alcoholism, manic-depressive psychosis, various forms of neurosis, and tabacosis

The method was carried out mainly in same way as in Example 1.

Male patient D., 42 has been addicting to alcoholic beverages since the age of 24. At the age of 35 the patient exhibited the initial signs of abstinence that affected badly patient's working capacity. He passed a treatment course in addictology clinics many times. However, alcohol addiction did not disappear after taking such treatment courses; but a dread of addiction remained, which determined the duration of remission period, its maximum duration did not exceed half a year. In the period immediately preceding the treatment by microwave resonance therapy the nature of the patient's alcohol addiction changed, i.e., the drinking bouts lasting 5 to 7 days occurring at a month interval. The patients was admitted to the clinic in the full swing of a bout, the diagnosis was one of stage II chronic alcoholism; he exhibited abstinence syndrome.

The patient was exposed to the effect of electromagnetic radiation within the wavelength range of 4.8 to 5.5 mm. The spectral density of the radiation power ranged within $10^{-12}$ and $10^{-18}$ W/Hz, the duration of a single treatment session, within 15 and 25 minutes. The place of application of said radiation - the following biologically active points: 'tsu-lee-pang', auricular pulmonary point P23, and 'he-gu'. During the initial four sessions the therapeutic action was applied to the point 'tsu-lee-pang', during further five sessions (5th to 9th), to the auricular pulnomary P23. Once the patient had developed an indifferent attitude towards alcohol, the therapeutic action was applied to the point 'he-gu'. A total of 14 sessions were performed.

Before the last session the patient developed an aversion for the smell of alcohol, accompanied by objectionable somatic and vegetative reactions.

Treatment of patients suffering from chronic alcoholism gave the following results: in 18 patients (out of 28) duration of therapeutic remission exceeded half a year, in 2 patients it exceed one year, whereas in 3 patients no clinical effect was detected. Control of the treatment efficiency has been performed using clinical method.

Treatment of patients with manic-depressive psychosis was instrumental in obtaining a considerable clinical effect, that is, restoration of working capacity.

Patients suffering from chronic alcoholism or tabacosis exhibited more prolonged period of abstinence from alcohol and tobacco.

Therapy of neurotic states made it possible to obtain a clinical effect in about 60 percent of 13 patients who passed the treatment course.

Therapy of tabacosis gave a 75-percent efficiency of treatment.

Industrial Applicability

The present invention can find application for treatment of patients suffering from chronic diseases of the lung, locomotorium infantile cerebral palsy, disturbances of the emotional-motivation field, and other diseases.

Claims

1. A device for microwave resonance therapy, comprising a source of the EHF-band electromagnetic oscillations, whose output is connected to a radiating antenna (2), CHARACTERIZED in that used as a source (1) of electromagnetic oscillations is a source of continuous frequency spectrum oscillations in a therapeutically effective frequency band having a spectral power density of from $10^{-6}$ W/Hz to a value approximating the quantum limit.

2. A device as claimed in Claim 1, CHARACTERIZED in that use is herein made of the source (1) of oscillations having a spectral density of electromagnetic radiation within $10^{-16}$ and $10^{-18}$ W/Hz.

3. A device as claimed in Claims 1 and 2, CHARACTERIZED in that used as a source of electromagnetic oscillations is a noise generator (1).

4. A device as claimed in Claim 3, CHARACTERIZED in that the noise generator (1) comprises a supply voltage shaper connected to the input of an active element (12), and a means (15) for channelling of electromagnetic energyemitted by the active element (12), while the radiating antenna (2) is connected to the output of said means (15) for channelling of electromagnetic energy.

5. A device as claimed in Claim 4, CHARACTERIZED in that the means (15) for channelling of electromagnetic energy is in fact a wave guide adapted for low-pass filtering of electromagnetic radiation.

6. A device as claimed in Claim 4, CHARACTERIZED in that it comprises additionally a waveguide transformer (16), whereby communication is established between the means (15) for channelling of electromagnetic energy and the radiating antenna (2).

FIG.1

FIG.3

FIG.2

EP 0 419 662 A1

# INTERNATIONAL SEARCH REPORT

International Application No PCT/SU 89/00099

| I. CLASSIFICATION OF SUBJECT MATTER (if several classification symbols apply, indicate all) * |
|---|

According to International Patent Classification (IPC) or to both National Classification and IPC

IPC⁵  A61H 39/00, A61N 5/02, 1/40

## II. FIELDS SEARCHED

Minimum Documentation Searched ⁷

| Classification System | Classification Symbols |
|---|---|
| IPC⁴ | A61H 39/00, A61N 1/40, 5/02 |

Documentation Searched other than Minimum Documentation
to the Extent that such Documents are Included in the Fields Searched ⁸

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| A | Elektronnaya promyshlennost, Nauchno-tekhnichesky sbornik No 8 (176), 1988 <br><br> "Ministerstvo elektronnoi promyshlennosti SSSR" (Moscow) pages 20-23 | 1-2 |
| A | Elektronnaya promyshlennost, Nauchno-tekhnichesky sbornik No. 2 (170) 1988 <br><br> "Ministerstvo elektronnoi promyshlennosti SSSR" (Moscow), page 53 | 1-2 |
| A | Elektronnaya promyshlennost, Nauchno-tekhnichesky sbornik No. 1 (159) 1987 <br><br> "Ministerstvo elektronnoi promyshlennosti SSSR" (Moscow) pages 31-33 (cited in the description) | 1-2 |

* Special categories of cited documents: ¹⁰
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art.
"&" document member of the same patent family

## IV. CERTIFICATION

| Date of the Actual Completion of the International Search | Date of Mailing of this International Search Report |
|---|---|
| 21 November 1989 (21.11.89) | 10 January 1990 (10.01.90) |

| International Searching Authority | Signature of Authorized Officer |
|---|---|
| ISA/SU | |